Europäisches Patentamt

European Patent Office

Office européen des brevets

(11) Publication number: **0 464 297 B1**

# EUROPEAN PATENT SPECIFICATION

(45) Date of publication of patent specification: **17.05.95**   (51) Int. Cl.⁶: **A61K 35/78**, A61K 7/00, C07F 9/10

(21) Application number: **90830307.6**

(22) Date of filing: **05.07.90**

(54) **Complexes of neolignane derivatives with phospholipids, the use thereof and pharmaceutical and cosmetic formulations containing them.**

(43) Date of publication of application:
**08.01.92 Bulletin 92/02**

(45) Publication of the grant of the patent:
**17.05.95 Bulletin 95/20**

(84) Designated Contracting States:
**AT BE CH DE DK ES FR GB GR IT LI LU NL SE**

(56) References cited:
**EP-A- 0 209 038      EP-A- 0 250 953**
**EP-A- 0 275 005      EP-A- 0 275 224**
**EP-A- 0 283 713      GB-A- 2 184 353**

(73) Proprietor: **INDENA S.p.A.**
**Via Ripamonti, 99**
**I-20141 Milano (IT)**

(72) Inventor: **Bombardelli, Ezio**
**Via Ripamonti, 99**
**I-20141 Milano (IT)**
Inventor: **Patri, Gianfranco**
**via Ripamonti, 99**
**I-20141 Milano (IT)**
Inventor: **Pozzi, Roberto**
**via Ripamonti, 99**
**I-20141 Milano (IT)**

(74) Representative: **Bianchetti, Giuseppe**
**Studio Consulenza Brevettuale,**
**Via Rossini, 8**
**I-20122 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to complexes of extracts from Krameria triandra Ruiz et Pav. and other plants of the Eupomatia genus, as well as some phenol constituents thereof of neo-lignane or nor-neolignane nature, with phospholipids; and to pharmaceutical compositions containing them.

The extracts from roots of Krameria triandra had been used widely in the past as medicaments, thanks to both the astringent property thereof, due to the presence of Ratania-tannin, and the antiinflammatory, antidiarrhoeal and antibacterial properties connected with other components; some of those characteristics are reported on Martindale, "The extrapharmacopeia", 28th Ed. (1982) and on other documents (Canizzaro, Boll. Soc. Ital. Biol. Sperim., 1, 22, 1964; and V. Hoppe, Drogenkunde Bdl Walter De Gruyter ed., 1975). Two components of neolignane nature have been described as sunscreens for possible cosmetic use (Stahl, Planta Med., 42, 144, 1981). The first remarks on the medicinal properties of said plant go back to the Peruvian people empiricism, which used the roots for the oral cavity hygiene ("raiz para los dientes"), which use spread rapidly also in other countries in more recent times.

British patent 2.184.353A claims the antibacterial and antimycotic properties of extracts from Krameria triandra and of some components, called Ratania-phenols, for which the use by the topical route is envisaged for the treatment of acne, dermatomycosis and decubitus ulcers attributable to infections caused by aerobic and anaerobic strains. A renewed interest in Krameria triandra derivatives came out from Italian preliminary searches, the results of which, related to the antibacterial and antimycotic activities, were reported in the 1st Int. Symposium on Organic Chemistry of Medicinal Natural Products, Shanghai, and also in a degree thesis at the University of Milan.

From the above cited literature data, the antibacterial and antimycotic activities turn out to be essentially related to Ratania-phenols of neolignane nature; said compounds showing in vitro a remarkable antimicrobial activity which, as we could ascertain, almost completely disappears after administration of the compounds by the systemic route, since the phenol groups easily undergo oxidation, or other enzyme degradations can occur. Topical treatments with total or purified extracts also show to a considerable degree the same drawbacks of the systemic route, thus decreasing the effectiveness of the active principles.

Now, it has surprisingly been found that, by reacting these substances with phospholipids in aprotic solvents, to generate the lipophilic complexes disclosed hereinafter, the same antimicrobial activities of the free active principles can be obtained in vitro, with a simultaneous remarkable improvement in the in vivo antimicrobial, antiinflammatory and antiradical activities.

In order to prepare the above mentioned complexes, either the main components of the Ratania-phenol class, such as Eupomatenoid 6 or the compound 2(2,4-dihydroxyphenyl)-5-propenylbenzofuran, which is among the main responsibles for the biological activity, or purified extracts standardized in these active principles have been used, the latter being prepared chiefly by extracting the roots with chlorinated solvents such as methylene chloride, chloroform, dichloroethane, etc. and partitioning the concentrate between an aliphatic alcohol and an aliphatic or aromatic hydrocarbon. Generally the roots are extracted with methylene chloride, the percolate is concentrated to small volume and the residue is dissolved in 90% aqueous methanol and counter-extracted with n-hexane; the methanol phase, after appropriate dilution with water, is counter-extracted with a chlorinated solvent which extracts the active principles; the chlorinated organic phase is concentrated to small volume and the residue is insolubilized with n-hexane.

According to a preferred object of the present invention, in order to maintain the stability of the active principles during the extraction processes, the finely ground roots are extracted with carbon dioxide under hypercritical conditions, working at a temperature of 40°C and under a pressure of 120 bars; after the extraction under said conditions, the vegetable material is further extracted with carbon dioxide added with acetone, at a temperature of 45°C and under a pressure of 200 bars; the residue from this second extraction, after evaporating off the gas, is dissolved in methylene chloride, dehydrated, decolorized on charcoal and insolubilized in hexane.

From the above prepared extracts, the single pure components can be isolated, using chromatography techniques on silica gel, working on the per se products or, preferably, on the acetyl derivatives thereof, which are subsequently deprotected under controlled conditions.

The above cited extracts contain about 50% Eupomatenoid 6 and 25% 2(2,4-dihydroxyphenyl)-5-propenylbenzofuran, as determined by gas chromatography or by HPLC. These substances, at the pure state, and the extracts containing them have been tested in vitro for the antiradical, antibacterial and antimycotic activities thereof, both as the free compounds and as the complexes thereof, and in vivo for the antiinflammatory and antimicrobial activities.

The obtained data are reported in Tables I, II and III.

The _in_ _vitro_ antimicrobial activity (M.I.C. expressed in mcg/ml) was evaluated in agar culture medium Isosensitest agar Oxoid on Gram-positive and Gram-negative microorganisms from both collection and hospital isolation; Difco blood agar was used for streptococci, whereas Sabouraud maltose agar Difco was used for yeasts, moulds and dermatophytes, by incubating the various inocula according to standard procedures. The results are reported in the following Table I.

Table I

Antimicrobial activity of Krameria active principles and of the related complexes.

| Microorganism | M.I.C. µg/ml | | | | | |
|---|---|---|---|---|---|---|
| | I | II | III | IV | V | VI |
| Staphylococ.aureus Smith | 0.012 | 0.78 | 1.56 | 3.12 | 1.56 | 0.78 |
| Staphylococ.aureus 9144 | 0.024 | 0.78 | 1.56 | 1.56 | 1.56 | 3.12 |
| Staphylococ.aureus F2*G | 50 | 0.78 | 1.56 | 3.12 | 1.56 | 3.12 |
| Staphylococ.aureus 6538P | 0.012 | 0.78 | 1.56 | 3.12 | 1.56 | 3.12 |
| Staphylococ.aureus OSCB* | 3.12 | 0.78 | 0.78 | 3.12 | 1.56 | 1.56 |
| Staphylococ.aureus FBF* | 0.78 | 0.78 | 1.56 | 1.56 | 1.56 | 1.56 |
| Streptococ.pyogenes 68 | 0.012 | 0.39 | 0.78 | 3.12 | 3.12 | 1.56 |
| Streptococ.pyogenes 68/24 | 0.012 | 0.39 | 0.78 | 3.12 | 0.65 | 0.65 |
| Streptococ.faecalis 6057 | 0.19 | 1.56 | 3.12 | 3.12 | 6.25 | 6.25 |
| Streptococ.faecalis 99/85 | 0.39 | 3.12 | 6.25 | 6.25 | 6.25 | 6.25 |
| Streptococ.salivarius 71/24 | 0.78 | 6.25 | 0.78 | 12.5 | 1.56 | 12.5 |
| Streptococcus. mutans 60/21 | 0.012 | 0.78 | 0.78 | 1.56 | 1.56 | 3.12 |
| Streptococ.pyogenes 65/57 | 0.19 | 0.78 | 0.78 | 0.78 | 1.56 | 6.25 |
| Streptococ.mitis 77/231 | 0.78 | 1.56 | 1.56 | 1.56 | 0.78 | 3.12 |
| Sarcinia lutea 9341 | 0.012 | 0.39 | 0.78 | 0.39 | 1.56 | 3.12 |
| Escherichia coli 120 | 0.39 | >200 | 50 | >200 | 100 | 100 |
| Salmonella typhi 6/12 | 0.19 | >100 | 25 | >100 | 50 | >100 |
| Salmonella enteritidis | 0.78 | 200 | 50 | >200 | 100 | >100 |
| Neisseria meningitidis | 0.012 | 1.56 | 0.78 | 1.56 | 1.56 | 3.12 |
| Klebsiella pneumonie | >200 | >200 | 12.5 | >200 | 12.5 | >100 |

3

Table I (continued)

| Microorganism | M.I.C. µg/ml | | | | | |
| --- | --- | --- | --- | --- | --- | --- |
| | I | II | III | IV | V | VI |
| Candida albicans G1 | 3.12 | 6.25 | 25 | 6.25 | 6.25 | >200 |
| Aspergillus niger | 6.25 | 6.25 | 12.5 | 6.25 | 12.5 | 100 |
| Trichophyton mentag. | 0.39 | 1.56 | 1.56 | 1.56 | 1.56 | 25 |
| Trichophyton tonsurans | 0.19 | 0.78 | 1.56 | 1.56 | 0.78 | 12.5 |
| Microsporum canis L/55 | 0.78 | 1.56 | 0.78 | 3.12 | 1.56 | 12.5 |

Substances

I = ampicillin for bacteria and miconazole for fungi.

II = Eupomatenoid 6.

III = 2(2,4-dihydroxyphenyl)-5-propenylbenzofuran.

IV = Complex of Eupomatenoid with phosphatidylcholine.

V = complex of 2(2,4-dihydroxyphenyl)-5-propenylbenzo-
     furan whit phosphatidylcholine.

VI = Standardized Krameria extract.

The antiinflammatory activity by the topical route was evaluated in the mouse by the Croton oil test, according to the procedures reported in Agents and Actions, 17, 347-49, 1985. The results are summarized in the following Table II.

4

Table II

| Antiinflammatory activity of the Krameria extract, of the active components thereof and of the related complexes in the Croton oil test in the mouse, at the maximum edema (6 hours). | | | |
|---|---|---|---|
| Substances | Dose/µg/ear | Edema mg | % reduction |
| Controls | --- | 7.4±0.2 | --- |
| Eupomatenoid 6 | 56<br>28 | 1.5±0.2<br>4.5±0.1 | 78.5 **<br>40.2 |
| Ratania-phenol* | 56<br>28 | 1.9±0.2<br>4.7±0.2 | 73.7 **<br>34.8 |
| Krameria extract | 70 | 1.8±0.3 | 75.0 ** |
| Phospholipid ° | 157<br>78 | 6.5±0.3<br>6.2±0.2 | 10.2<br>14.0 |
| Eupomatenoid/complex 1M:1M | 96 | 1.9±0.2 | 73.7 ** |
| Krameria extr./Complex 1p:2p(w/w) | 105 | 1.6±0.3 | 78.2 ** |

\* 2(2,4-dihydroxyphenyl)-5-propenylbenzofuran.
° Distearoylphosphatidylcholine.
°° P<0.01 Student t.

The antiradical activity was evaluated using DPPH as the competitor, according to procedures described in literature. Following Table III reports the obtained results.

Table III

| Antiradical activity of a Krameria standardized extract and of two components thereof. | | |
|---|---|---|
| Substances | Conc.µg/ml | % destroyed DPNH |
| Eupomatenoid 6 | 6.6<br>2.8 | -75.7<br>-40.8 |
| Ratania-phenol* | 6.6<br>2.8 | -80.5<br>-46.4 |
| Krameria extract | 10<br>5 | -72.4<br>-41.6 |
| Quercetin | 6.2 | -68.2 |

\* 2(2,4-dihydroxyphenyl)-5-propenylbenzofuran.

In order to prepare the complexes with phospholipids, pure soy phosphatidylcholine or standardized mixtures of vegetable phospholipids with titres from 90% to 100% or pure synthetic phospholipids having saturated or unsaturated acyl chains and choline or ethanolamine as the basic portion, were selected as the complexing agents.

The formation of said complexes is ascertained by means of $^1$H-NMR, $^{13}$C-NMR, $^{31}$P-NMR spectroscopies and solubility tests. As regards solubility, substances which are insoluble in aprotic solvents such as aromatic hydrocarbons, become easily soluble after complexation; in the $^1$H-NMR spectrum the signals of complexed substances undergo a strong broadening, so as they can no more be evidenced in the spectra; in the $^{13}$C-NMR spectrum the signals of the complexed substance, as well as those of the choline and glyceryl portions of the phospholipid, can no more be recorded; the phosphorus nucleus itself undergoes a band broadening, which indicates it is involved in the complex formation; in both the $^1$H and the $^{13}$C spectra, substantially only the lipid chain signals appear, even showing some immobilization. The above data prove the interaction between the phospholipid polar head and the active sites of the complex, whereas the lipid chains are not involved, since they are free to rotate and can wind the complexed molecule giving it a marked lipophilia.

This winding clearly results in a particular steric configuration when the complex is dispersed in an aqueous medium, giving stability to the molecules themselves.

The complexes of the present invention are insoluble in water and cannot form in this medium, therefore associations of the same products with phospholipids or liposomal preparations thereof do not give the same biological results; the complexes of the invention, due to dipolarity reasons, in aqueous medium give rise to micellar microdispersions remarkably similar to the liposome structures. The novel structures according to the invention show, in comparison with the same structures in the free form, a different in vivo bioavailability which involves an increase in the specific activity as well as a longer lasting action by the topical route (skin and accessible mucosae).

For this class of substances, phospholipids are a selective carrier allowing the molecules to have both an improved crossing of the horny layer of the skin and a better interaction with cell and bacterial walls. The molecules in the free form are restored and retained in the action site for a longer time. The presence of a surfactant, i.e. the phospholipid, in the molecule, allows to obtain a higher adhesion of the product itself to the surfaces it comes into contact with. This aspect is of paramount importance in cosmetic and pharmaceutical formulations intended for the treatment of the oral cavity, in which contact times are of course very short; the opportunity to increase the adhesion of the product to mucosae or to the tooth surface and to make the various molecules interact with cell structures is of basic practical importance and it is an object of the present invention.

The dosages of the complexes of the invention can range widely, depending on the intended therapeutical purposes, and they can vary from 0.001 to 1%. The complexes of the present invention can be incorporated in the traditional pharmaceutical forms, such as ointments, fluid and thick O/W or W/O emulsions, solutions, chewable tablets, dusting powders, or in form of plasters or medicated gauzes. Said preparations can be used in humans or in animals for the treatment of superficial infected inflammatory processes, in torpid sores and in all the phlogistic conditions of the oral cavity; more particularly, as far as the oral cavity is concerned, they are an effective protection against the formation of dental plaque, when administered in form of medicated toothpaste, collutory or gel. In the cosmetic field, said complexes can be used for the treatment of acne and as deodorant, antidandruff and personal hygiene products.

EXAMPLE 1

Preparation of a standardized Krameria triandra extract.

10 kg of finely ground roots of Krameria triandra are extracted 4 times under mild reflux and nitrogen atmosphere with 4 volumes of methylene chloride; the combined extracts are concentrated to small volume under normal pressure. The concentrate is taken up into 5 l of 90% aqueous methanol and the solution is extracted 3 times with 1.5 l of n-hexane; the hexane phase is discarded since it contains no active principles whereas the methanol phase is concentrated to 1.2 l; this concentrate is extracted 3 times with 1.5 l of methylene chloride. The hydroalcoholic phase is discarded, whilst the chloromethylene extracts are pooled and treated with 25 g of decolorant charcoal in the presence of anhydrous sodium sulfate as the dehydrating agent; the decolorized chloromethylene solution is concentrated to small volume and the concentrate is poured into 1.5 l of hexane, under strong stirring. The precipitate is filtered and dried under vacuum overnight at 40°C, to obtain 300 g of a product containing about 50% Eupomatenoids and 27% 2-(2,4-dihydroxyphenyl)-5-propenylbenzofuran.

EXAMPLE 2

Preparation of Eupomatenoid 6 and 2-(2,4-dihydroxyphenyl)-5-propenylbenzofuran.

100 g of an extract prepared according to the procedures reported in Example 1 are chromatographed on a column containing 1.5 kg of silica gel, which has previously been equilibrated with a toluene/ethyl acetate 9:1 mixture, eluting the products with the same solvent mixture; the fractions showing a similar composition by thin layer chromatography are pooled and concentrated to dryness. The more abundant fraction, containing Eupomatenoid 6 (54 g) is crystallized from an hexane-isopropyl ether mixture, thus recovering the pure compound; analogously, the fraction containing the benzofuran derivative is crystallized so as to isolate also this compound, the physico-chemical and spectroscopical characteristics of which are the same as those reported in literature.

EXAMPLE 3

Preparation of a purified Krameria extract by means of carbon dioxide under supercritical conditions.

1.5 kg of finely ground roots of Krameria are extracted continuously in a suited reactor, fitted with heating and cooling mantle, with carbon dioxide under supercritical conditions, in two steps. A first step is carried out at a temperature of 35°C and under a pressure of 110 bars; in the evaporator temperature and pressure are 25°C and 50 bars, respectively. Two hours after, the extracted material is unloaded from the condenser and carbon dioxide containing 1.5% acetone is circulated in the extractor. The first extract, containing only lipophilic substances with no activity, is discarded, whereas the second extract is collected, which is obtained by increasing temperature to 45°C and pressure to 200 bars. The collected extract in the condenser is dissolved in isopropyl ether, the solution is dried over sodium sulfate and subsequently concentrated to small volume. The concentrate is poured into n-hexane : an abundant precipitate forms which, upon drying, weighs 46 g and has the same composition as that of the product obtained in Example 1, but it has a markedly lighter colour.

EXAMPLE 4

Preparation of the complex of the standardized extract of Krameria with soy phosphatidylcholine.

1.5 g of a purified Krameria triandra extract are dissolved together with 3 g of soy phosphatidylcholine in 30 ml of methylene chloride and heated to mild reflux for 2 hours; the chloromethylene solution is concentrated to dryness under vacuum at 30°C until the solvent is completely evaporated off. The $^1$H-NMR spectrum of the obtained product shows protonic signals at 0.8 ppm of aliphatic methyls of the lipid chains and $CH_2$ signals between 1.5 and 2.8 ppm, a very broad $N-CH_3$ signal at about 3 ppm, signals of hydrogens on the double bond of aliphatic chains at 5.5 ppm and very broad, unresolved signals of aromatic protons of the Ratania-phenols at 6-8 ppm. In the $^{13}$C-NMR spectrum, the signals characteristics of Ratania-phenols between 102 and 135 ppm are absent or unresolved, and the signals of the carbons of phospholipid glycerin portion are also absent or extremely broadened, as well as those of the choline portion.

EXAMPLE 5

Preparation of the complex of Eupomatenoid 6 with soy phosphatidylcholine.

2.8 g (0.01 mole) of Eupomatenoid 6 are dissolved together with 7.8 g (0.001 mole) of pure soy phosphatidylcholine in 30 ml of dioxane free from peroxides and the solution is freeze-dried; 10.5 g of a white product are obtained, having m.p. 98-101°C and spectroscopic characteristics in conformity with those of a complex. $^1$H-NMR ($C_6D_6$ solution): strong broadening of aromatic protons at 6-8 ppm; broadening of the $N-CH_3$ signal at 3.3 ppm. The characteristic signals of lipid chains appear at 0.5-2 ppm. $^{13}$C-NMR ($C_6D_6$ solution) : absence of the signals of carbons of Eupomatenoid at 105-140 ppm and also of the lipid signals at 60-80 ppm.

EXAMPLE 6

Preparation of the complex of 2-(2,4-dihydroxyphenyl)-5-propenylbenzofuran with distearoylphosphatidylcholine.

2.78 g of 2-(2,4-dihydroxyphenyl)-5-propenylbenzofuran are dissolved in 50 ml of dioxane together with 7.95 g of distearoylphosphatidylcholine and heated under strong stirring for 3 hours at 50°C, under argon atmosphere. When dissolution is completed the whole is freeze-dried, to obtain a white-beige product having spectroscopic characteristics corresponding to those of a complex.

EXAMPLE 7

Preparation of a standardized extract of Eupomatia laurina.

10 kilograms of finely ground roots of Eupomatia laurina are extracted under weak reflux in a nitrogen atmosphere with 4 volumes of methylene chloride for four times and the pooled extracts are concentrated to a small volume at ordinary pressure. The concentrate is then dissolved in 2 l of 90 per cent aqueous methanol and the resulting solution is extracted three times with 1.5 l of petroleum ether.

The petroleum ether phase not containing the active principles is discarded and the methanolic phase is concentrated to 0.9 l. The latter concentrate is diluted with 300 ml of water and extracted three times with 0.8 l of methylene chloride. The hydroalcoholic phase is discarded and the pooled methylene chloride extracts are treated with 25 g of decolorizing charcoal in the presence of anhydrous sodium sulphate as dehydrating agent. The decolorized methylene chloride solution is concentrated to a small volume and the concentrate is poured under vigorous shaking into 1 l of hexane. After filtration of the precipitate and overnight drying under vacuum at 40°C, 190 g are obtained of a product which contains as a major component Eupomatenoide 6.

EXAMPLE 8

Preparation of the complex between the standardized extract of Eupomatia laurina and soy-bean phosphatidylcholine.

20 g of purified extract of Eupomatia laurina are dissolved together with 40 g of soy-bean phosphatidylcholine in 300 ml of methylene chloride and heated under weak reflux for 2 h. The methylene chloride solution is concentrated to dryness under vacuum at 30°C until the solvent has been completely removed. The 1H-NMR spectrum of the final product shows protonic singnals at 0.8 ppm originating from the aliphatic methyl group of the lipidic chains, $CH_2$ signals between 1.5 and 2.8 ppm, a very broadened $N-CH_3$ signal at around 3.4 ppm, signals from hydrogens on double bonds of aliphatic chains at 5.5 ppm and very broadened unresolved signals of the aromatic protons of phenols Eupomatenoide 6 like substances between 6.2 and 8 ppm. In the carbon spectra it is remarkable the absence or the lack of resolution of the typical signals of the Eupomatenoide like substances between 102 and 135 ppm and the absence or the extreme broadening of the signals of the carbons belonging to the glycerine and choline portions of the phospholipid. This complex is freely soluble in the chorinated solvents and in the vegetal oils.

EXAMPLE 9

Preparation of a toothpaste containing the complex of the standardized Krameria extract.

| 100 g contain : | |
|---|---|
| Complex of Krameria standardized extract | 0.1 g |
| Sodium carboxymethyl cellulose m.v. | 1.0 " |
| Sorbitol 70% | 20.0 " |
| Glycerin | 18.0 " |
| Colloidal silica | 15.0 " |
| Titanium dioxide | 2.5 " |
| Maize starch | 2.0 " |
| Sodium laurylsulfate | 2.0 " |
| Sodium laurylsarcosinate 30% s. a. | 1.0 " |
| Flavouring composition | 1.0 " |
| Purified water     q.s.to | 100.0 " |

EXAMPLE 10

Preparation of chewable tablets containing the complex of Krameria standardized extract.

| Each 1 g tablet contains : | |
|---|---|
| Complex of Krameria standardized extract | 5.0 mg |
| Methyl cellulose m.v. | 2.0 " |
| Colloidal silica | 8.0 " |
| Magnesium stearate | 10.0 " |
| Mannitol | 250.0 " |
| Glycamil (monoammonium glycyrrhizinate) | 5.0 " |
| Flavours | 20.0 " |
| Saccharose (compressible) | 700.0 mg |

EXAMPLE 11

Preparation of a medicated cream containing the complex Eupomatenoid/phosphatidylcholine.

| 100 g of an O/W emulsion contain : | |
|---|---|
| Complex Eupomatenoid/phosphatidylcholine | 0.1 g |
| PEG-8 $C_{12-18}$ alkyl ester | 10.0 " |
| Glyceryl stearate and PEG-8 stearate | 2.5 " |
| Isopropyl myristate | 7.0 " |
| Preservative | 0.1 " |
| Glycerin | 5.0 " |
| Perfumed composition | 0.5 " |
| Purified water     q.s.to | 100.0 g |

EXAMPLE 12

Preparation of an aqueous gel containing the complex of the Krameria standardized extract

| 100 g of gel contain : | |
|---|---|
| Complex of standardized Krameria extract | 0.1 g |
| PEG-6 caprylic/capric glycerides | 10.0 " |
| Ethoxylated oleyl alcohol | 5.0 " |
| Carboxyvinyl polymer | 1.5 " |
| Preservatives | 0.3 " |
| Perfumed composition | 0.2 " |
| Sodium hydroxide | 0.3 " |
| Purified water     q.s.to | 100.0 " |

EXAMPLE 13

Preparation of a lipogel containing the complex of the Krameria standardized extract.

| 100 g of gel contain : | |
|---|---|
| Complex of standardized Krameria extract | 0.1 g |
| PEG-45 dodecylglycole copolymer | 4.0 " |
| Hydroxystearate | 4.0 " |
| Polyisoprene | 37.5 " |
| Isopropyl myristate | 38.9 " |
| Silicone oil 350 cps | 3.5 " |
| Hydrogenated castor oil | 3.5 " |
| Colloidal silica | 3.0 " |
| Polysorbate 80 | 3.0 " |
| Perfumed composition | 0.5 g |

EXAMPLE 14

Preparation of a dusting powder containing the complex of the Krameria standardized extract.

| 100 g of powder contain : | |
|---|---|
| Complex of Krameria standardized extract | 0.1 g |
| Colloidal silica | 2.0 " |
| Micronized hydrogenated castor oil | 49.0 " |
| Maize starch | 48.9 " |

**Claims**

**Claims for the following Contracting States : AT, BE, CH, DE, DK, FR, GB, IT, LI, LU, NL, SE**

1.  Complexes of lipophilic extracts from plants of Krameria or Eupomatia genus, with natural or synthetic phospholipids.

2.  Complexes of Ratania-phenols with natural or synthetic phospholipids.

3.  Complexes of Eupomatenoid 6 with natural or synthetic phospholipids.

4.  Complexes of 2-(2,4-dihydroxyphenyl)-5-propenylbenzofuran with natural or synthetic phospholipids.

5.  Complexes as claimed in the preceding claims, in which phospholipids are selected from soy phosphatidylcholine, soy phosphatidylethanolamine, natural phospholipids having titres from 90 to 100% and pure synthetic phospholipids with saturated or unsaturated acyclic chains, having ethanolamine or choline as the basic portion.

6.  Pharmaceutical and cosmetic compositions having antiinflammatory, antibacterical, antimycotic and antiradical activities, containing as the active principles the complexes of claims 1-5.

**Claims for the following Contracting States : ES, GR**

1.  A process for the preparation of complexes of lipophilic extracts from plants of Krameria or Eupomatia genus or of Ratania-phenols contained therein with natural or synthetic phospholipids characterized in that the lipophilic extracts or the Ratania-phenols are reacted with phospholipids in aprotic solvents.

**2.** A process according to claim 1 in which phospholipids are selected from soy phosphatidylcholine, soy phosphatidylethanolamine, natural phospholipids having titres from 90 to 100% and pure synthetic phospholipids with saturats or unsaturated chains, having ethanolamine or choline as the basic portion.

**Patentansprüche**
**Patentansprüche für folgende Vertragsstaaten : AT, BE, CH, DE, DK, FR, GB, IT, LI, LU, NL, SE**

**1.** Komplexe lipophiler Extrakte aus Pflanzen der Sorten Krameria oder Eupomatia mit natürlichen oder synthetischen Phospholipiden.

**2.** Komplexe von Ratania-Phenolen mit natürlichen oder synthetischen Phospholipiden.

**3.** Komplexe von Eupomatenoid 6 mit natürlichen oder synthetischen Phospholipiden.

**4.** Komplexe von 2-(2,4-Dihydroxyphenyl)-5-propenylbenzofuran mit natürlichen oder synthetischen Phospholipiden.

**5.** Komplexe nach einem der vorhergehenden Ansprüche, bei denen die Phospholipide Sojaphosphatidylcholin, Sojaphosphatidylethanolamin, natürliche Phospholipide mit Titern von 90 - 100 % und reine synthetische Phospholipide mit gesättigten oder ungesättigten acyklischen Ketten mit Ethanolamin oder Cholin als Basis sind.

**6.** Pharmazeutische oder kosmetische Zusammensetzungen mit antientzündlichen, antibakteriellen, antimykotischen und antiradikalischen Aktivitäten, die als aktive Ingredienzien die Komplexe gemäß Ansprüchen 1 - 5 enthalten.

**Patentansprüche für folgende Vertragsstaaten : ES, GR**

**1.** Verfahren zur Herstellung von Komplexen aus lipophilen Extrakten aus Pflanzen der Sorten Krameria oder Eupomatia oder darin enthaltenen Ratania-Phenolen mit natürlichen oder synthetischen Phospholipiden, dadurch gekennzeichnet, daß die lipophilen Extrakte oder die Ratania-Phenole mit Phospholipiden in aprotischen Lösungsmitteln umgesetzt werden.

**2.** Verfahren nach Anspruch 1, wobei die Phospholipide Sojaphosphatidylcholin, Sojaphosphatidylethanolamin, natürliche Phospholipide mit Titern von 90 bis 100 % und/oder reine synthetische Phospholipide mit gesättigten oder ungesättigten Seitenketten, mit Ethanolamin oder Cholin als Basis, sind.

**Revendications**
**Revendications pour les Etats contractants suivants : AT, BE, CH, DE, DK, FR, GB, IT, LI, LU, NL, SE**

**1.** Complexes d'extraits lipophiles de plantes du genre *Krameria* ou *Eupomatia*, avec des phospholipides naturels ou synthétiques.

**2.** Complexes de Ratania-phénols avec des phospholipides naturels ou synthétiques.

**3.** Complexes d'Eupomaténoïde 6 avec des phospholipides naturels ou synthétiques.

**4.** Complexes de 2-(2,4-dihydroxyphényl)-5-propénylbenzofuranne avec des phospholipides naturels ou synthétiques.

**5.** Complexes tels que revendiqués dans les revendications précédentes, dans lesquels les phospholipides sont choisis parmi la phosphatidylcholine de soja, la phosphatidyléthanolamine de soja, les phospholipides naturels ayant des titres de 90 à 100 % et les phospholipides synthétiques purs à chaînes acycliques saturées ou insaturées, ayant de l'éthanolamine ou de la choline comme portion basique.

**6.** Compositions pharmaceutiques et cosmétiques ayant des activités anti-inflammatoires, antibactériennes, antifongiques et antiradicalaires, contenant comme principes actifs les complexes des revendications 1 à 5.

**Revendications pour les Etats contractants suivants : ES, GR**

1.  Un procédé de préparation de complexes d'extraits lipophiles de plantes du genre *Krameria* ou *Eupomatia ou de Ratania-phénols contenus dans celles-ci*, avec des phospholipides naturels ou synthétiques, caractérisé en ce qu'on fait réagir les extraits lipophiles des Rataniaphénols avec des phospholipides dans des solvants aprotiques.

2.  Un procédé selon la revendication 1, dans lequel les phospholipides sont choisis parmi la phosphatidylcholine de soja, la phosphatidyléthanolamine de soja, les phospholipides naturels ayant des titres de 90 à 100 % et les phospholipides synthétiques purs à chaînes acycliques saturées ou insaturées, ayant de l'éthanolamine ou de la choline comme portion basique.